(19) Europäisches Patentamt
European Patent Office
Office européen des brevets

(11) Publication number : **0 526 033 A1**

(12) # EUROPEAN PATENT APPLICATION

(21) Application number : **92306426.5**

(22) Date of filing : **14.07.92**

(51) Int. Cl.[5] : **C07D 401/12,** A61K 31/415, A61K 31/44

(30) Priority : **18.07.91 GB 9115534**

(43) Date of publication of application : **03.02.93 Bulletin 93/05**

(84) Designated Contracting States : **PT**

(71) Applicant : **FISONS plc**
**Fison House Princes Street**
**Ipswich Suffolk IP1 1QH (GB)**

(72) Inventor : **Ingall, Anthony Howard**
**53 Forest Road**
**Loughborough, Leicestershire LE11 3NW (GB)**

(74) Representative : **Gilholm, Stephen Philip**
**Fisons plc 12 Derby Road**
**Loughborough, Leicestershire LE11 0BB (GB)**

(54) **Benzimidazoles.**

(57) There are described compounds of formula I,

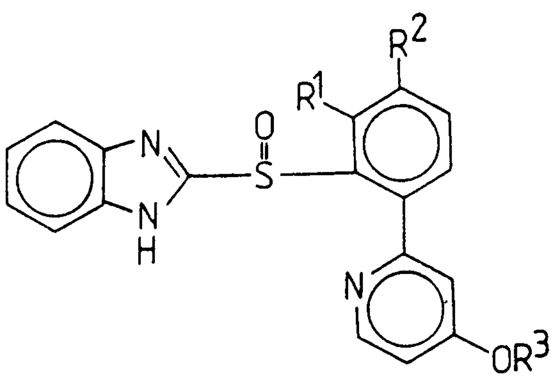

I

wherein one of $R^1$ and $R^2$ represents halogen or alkyl $C_{1-6}$, and the other represents hydrogen, $R^3$ represents alkyl $C_{1-6}$,
and pharmaceutically acceptable salts thereof.

Processes for making the compounds and pharmaceutical compositions containing them, e.g. for the treatment of conditions in which gastric acid has a pathological role, are also disclosed.

EP 0 526 033 A1

This invention relates to novel compounds, methods for their preparation and pharmaceutical compositions containing them.

European Patent Applications No. 220053 and 262845 disclose a number of benzimidazole derivatives and their use as inhibitors of gastric acid secretion.

We have now found a small group of benzimidazole derivatives which exhibit significant advantages over those known from the prior art.

According to the invention, there are provided compounds of formula I,

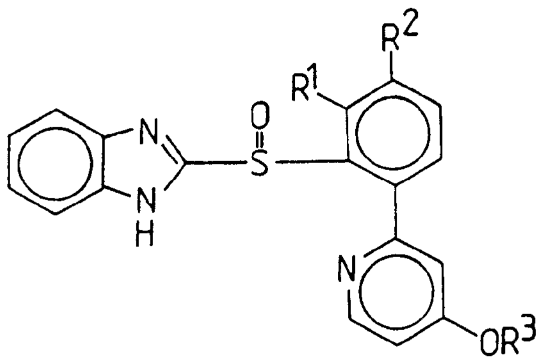

I

wherein one of $R^1$ and $R^2$ represents halogen or alkyl $C_{1-6}$ and the other represents hydrogen, and $R^3$ represents alkyl $C_{1-6}$,
and pharmaceutically acceptable salts thereof.

According to the invention we also provide a process for the production of a compound of formula I, or a pharmaceutically acceptable salt thereof, which comprises selective oxidation of a corresponding compound of formula II,

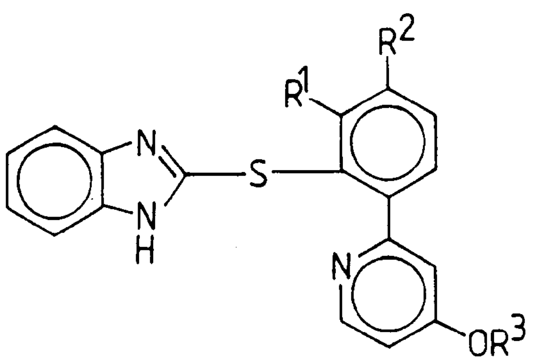

II

in which $R^1$, $R^2$ and $R^3$ are as defined above, and where necessary or desired converting the resulting compound of formula I to a pharmaceutically acceptable salt thereof, or vice versa.

The oxidation is preferably carried out in a solvent which is inert to the reaction conditions, for example, a polar, aprotic solvent. Suitable solvents include dichloromethane, chloroform, ethyl acetate, methanol, ethanol, water or a mixture thereof. The reaction is preferably carried out at less than room temperature, e.g. -20° to + 10° C. Suitable oxidising agents include peracids, e.g. m-chloroperbenzoic acid, magnesium monoperoxyphthalate, hydrogen peroxide or t-butylhydroperoxide in the presence of a suitable catalyst, e.g. vanadylacetylacetonate. The oxidation may, if desired, be carried out in the presence of a base, for example, lithium carbonate or sodium carbonate.

Compounds of formula II may be prepared by reacting a compound of formula III,

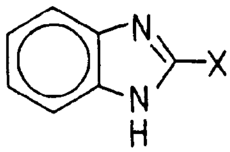

III

in which X is a good leaving group, with a compound of formula IV,

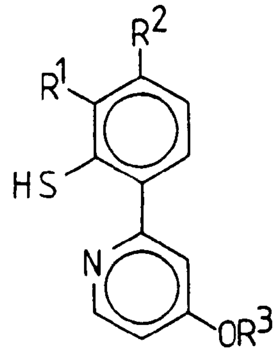

IV

in which $R^1$, $R^2$ and $R^3$ are as defined above.

Good leaving groups that X may represent include halogen, e.g. chlorine. The coupling reaction is preferably carried out in a solvent which is inert to the reaction conditions, e.g. isopropanol. The reaction may, if desired, be acid catalysed, e.g. using acetic acid; or base catalysed, e.g. using potassium carbonate. Compounds of formula III are known *per se*. Compounds of formula IV may be prepared from compounds known per se using the methods and conditions described in the examples, as will be clear to those skilled in the art.

Pharmaceutically acceptable salts of the compound of formula I include salts of the acidic benzimidazole nucleus with suitable organic or inorganic bases, for example, ammonium; alkali metal, e.g. sodium and potassium; alkaline earth metal, e.g. calcium and magnesium; and alkylamino salts. Further pharmaceutically acceptable salts which may be mentioned include salts with suitable organic or inorganic acids, e.g. with a hydrohalic, sulphuric, alkanesulphonic, tartaric or citric acid.

The compound of formula I may be obtained in the form of a salt, conveniently a pharmaceutically acceptable salt. Pharmaceutically acceptable salts may be prepared by reacting the compound of formula I with an appropriate base or acid in the presence of a suitable solvent.

The compounds of formula I may exist in enantiomeric forms, all enantiomers, racemates and mixtures thereof are included within the scope of the invention. The various optical isomers may be isolated by separation of a racemic mixture of the compounds using conventional techniques, e.g. fractional crystallisation, or HPLC. Single enantiomers may also be prepared by the enantioselective oxidation of a compound of formula II using a selective oxidant, for example, a chiral oxaziridine. Alternatively the individual enantiomers may be made by reaction of the appropriate optically active starting materials under reaction conditions which will not cause racemisation.

By the term alkyl we mean straight, branched or cyclic saturated or unsaturated alkyl groups.

Halogens that $R^1$ and $R^2$ may represent include fluorine, chlorine and bromine.

We prefer compounds of formula I in which $R^1$ represents halogen or alkyl $C_{1-6}$ and $R^2$ represents hydrogen.

We particularly prefer compounds of formula I in which $R^1$ represents alkyl $C_{1-6}$, especially methyl.

We prefer compounds of formula I in which $R^3$ is methyl.

The compounds of formula I, and pharmaceutically acceptable salts thereof, are useful because they possess pharmacological activity in animals; in particular they are useful because they have cytoprotective properties, are useful in the treatment or prophylaxis of inflammatory situations and/or prevent or inhibit gastric acid secretion. The activity of the compounds of formula I may be demonstrated in the following screens:

a) Rabbit gastric gland test; T. Berglindh and K.J. Obrink, *Acta. Physiol. Scand.*, 1976, **96**, 150-159, and **97**, 401-414; and E. Fellenius *et al, Am. J. Physiol.*, 1982, **243**(6), G505-510.

b) Heidenhain pouch dog test; J. Rudick and T. Szabo, *Mount Sinai J. Med.*, 1976, **43**, 423-446; and A.W. Herling *et al, Pharmacology*, 1988, **36**, 289-297.

According to a further aspect of the invention we provide a compound of formula I, or a pharmaceutically

acceptable salt thereof, for use as a pharmaceutical.

The compounds of formula I, and salts thereof, are indicated for use in the prevention or inhibition of gastric acid secretion in those conditions in which gastric acid has a pathological role. The compounds of formula I are therefore indicated for the treatment of peptic ulcers. Other conditions which may be mentioned include duodenal, gastric, recurrent or stromal ulceration, ulcers induced by corticosteroid treatment or major surgery, dyspepsia, duodenitis, Zollinger-Ellison syndrome, reflux oesophagitis and the management of haemorrhage, e.g. from erosion of ulcers in the upper gastrointestinal tract, especially when a major blood vessel is not involved. The compounds may also be used to treat gastritis or dyspepsia, particularly when associated with administration of non-steroidal anti-inflammatory drugs, in the prophylaxis of gastrointestinal haemorrhage from stress ulceration in seriously ill or burned patients, in the prophylaxis of recurrent haemorrhage in patients with bleeding peptic ulcers, before general anaesthesia in patients at risk of acid aspiration syndrome (Mendelson's syndrome) and to reduce the chance of haemorrhage in patients with leukaemia, graft versus host disease or with severe hepatic failure. The above conditions may be treated whether or not they are associated with excess gastric acid secretion.

The compounds of formula I, and pharmaceutically acceptable salts thereof, are also indicated for use as cytoprotective agents, especially for the gastrointestinal tract, and can be utilized for the treatment or prevention of a non-gastric-acid-induced, non-traumatically-induced, non-neoplastic gastrointestinal inflammatory disease, for example, Crohn's disease, inflammatory bowel disease, infectious enteritis, colitis, ulcerative colitis, pseudomembranous colitis, diverticulitis, and allergenic and radiological inflammatory diseases.

The compounds of formula I, and salts thereof, are also indicated for use in the treatment or prophylaxis of inflammatory conditions in mammals, including man, especially those involving lysozymal enzymes. Conditions that may be specifically mentioned are: rheumatoid arthritis, gout, eczema, polyserositis and allergic alveolitis.

According to a further aspect of the invention we provide a method for the prevention or inhibition of gastric acid secretion, which comprises administration of an effective amount of a compound of formula I to a patient suffering from or susceptible to a condition in which gastric acid has a pathological role.

The compounds of formula I, and salts thereof, may also be used to treat cholera, paratyphus, tourist diarrhoea, toxin-induced diarrhoea and local gastric catarrh.

Patterns of therapeutic use which may be mentioned are:-

a) a high dose initially, for say 2-4 weeks, followed by lower-dose maintenance therapy after the condition has improved, e.g. the ulcer has healed;

b) as in a) above, but the maintenance therapy including another cytoprotective agent, e.g. a $PGE_2$ derivative;

c) combination therapy, using a low dose of the compound of the invention in association with a low, well-tolerated dose of another cytoprotectant and/or antacid; or

d) intermittent dosing, e.g. every second day or at weekends, may be appropriate as maintenance therapy.

For the above mentioned uses the dosage administered will, of course, vary with the compound or salt employed, the mode of administration and the treatment desired. However, in general, the compounds exhibit satisfactory activity in the test set out in *Am. J. Physiol.*, 1982, **243**(6), G505-510, when administered at concentrations of from $10^{-6}$M to $10^{-4}$M. For man the indicated total daily dosage is in the range of from about 1 mg to 3,000 mg, preferably 5 to 500 mg, and more preferably from about 10 mg to 200 mg, which may be administered in divided doses from 1 to 6 times a day or in sustained release form. Thus unit dosage forms suitable for administration comprise from about 1.0 mg to 600 mg of the compound admixed with a solid or liquid pharmaceutically acceptable diluent, carrier or adjuvant.

According to a further aspect of the invention there is provided a pharmaceutical composition comprising preferably less than 80% w/w and more preferably less than 50% w/w, of a compound of formula I, or a pharmaceutically acceptable salt thereof, in admixture with a pharmaceutically acceptable adjuvant, diluent or carrier.

Examples of such adjuvants, diluents and carriers are

for tablets and dragees - fillers e.g. lactose, starch, microcrystalline cellulose, dibasic calcium phosphate, talc and stearic acid; lubricants/glidants e.g. magnesium stearate and colloidal silicon dioxide; disintegrants e.g. sodium starch glycolate and sodium carboxymethylcellulose;

for capsules - pregelatinised starch or lactose;

for oral or injectable solutions or enemas - water, glycols, alcohols, glycerin, vegetable oils;

for suppositories - natural or hardened oils or waxes.

Compositions in a form suitable for oesophageal administration include tablets, capsules, dragees, liquid suspensions or solutions;

compositions in a form suitable for topical administration to the skin include creams, e.g. oil-in-water

emulsions, water-in-oil emulsions, ointments or gels;

The compounds may also be administered transdermally using a suitable vehicle base, e.g. in an ointment base, which may be incorporated into a patch for controlled delivery through the skin.

The compound of formula I, or the pharmaceutically acceptable salt thereof, preferably has a particle size mass median diameter of from 0.1 to 250 μm, more preferably 1.0 to 50 μm. The compound of such particle size may be made by grinding or milling followed if necessary by particle size classification using, for example, a sieve. The compositions may also contain suitable preserving, stabilising, and wetting agents, solubilizers, sweetening and colouring agents and flavourings. The compositions may, if desired, be formulated in sustained release form.

The compounds may, if desired, be co-administered with other pharmaceutically active compounds, for example, antacids; or antibacterials e.g. bismuth subcitrate, amoxycillin or metronidazole.

We prefer compositions which are designed to be taken by ingestion or rectally and to release their contents in the intestine. We particularly prefer compositions which will pass through the acidic parts of the gastrointestinal tract unaffected, e.g. enteric coated formulations. Enteric coated formulations usually take the form of tablets comprising the compound of formula I which are coated with a polymer which is insoluble in gastric media, but which hydrates and begins to dissolve in the duodenum and small intestine. Enteric coatings which may be mentioned include cellulose acetate phthalate, acrylate polymers e.g methacrylic acid copolymer types A and B, hydroxypropylmethylcellulose phthalate and polyvinyl acetate phthalate. Enteric coatings may be applied to tablets in the form of a solution in an organic solvent or as an aqueous dispersion. To improve the mechanical properties of the enteric coating it may be necessary to add a plasticiser to the solution or dispersion, suitable plasticisers include low molecular weight phthalate esters, for example, dibutyl phthalate, acetylated monoglycerides, tributyl citrate and triacetin.

According to a further aspect of the invention we provide the use of a compound of formula I, or a pharmaceutically acceptable salt thereof, in the manufacture of a medicament for the treatment or prophylaxis of a condition in which gastric acid has a pathological role.

The compounds of formula I, and pharmaceutically acceptable salts thereof, have the advantage that they are more readily absorbed, are more soluble, are less irritant to the gastrointestinal tract, have less toxic side effects, are more active, are more stable to gastric acid when administered by ingestion, or have other useful pharmacological properties, than compounds of the prior art.

The invention is illustrated, but in no way limited by the following Examples, in which temperatures are in degrees Celsius.

Example 1

2-[5-Fluoro-2-(4-methoxy-2-pyridinyl)-phenylsulphinyl]-1H-benzimidazole

a) O-(2-Bromo-5-fluorophenyl)-N,N-dimethylthiocarbamate

A mixture of 2-bromo-5-fluorophenol (26.21 g) in dry dimethylformamide (150 ml), anhydrous $K_2CO_3$ (28.42 g) and N,N-dimethyl thiocarbamoyl chloride (20.73 g) was stirred at room temperature for 18 hours, then anhydrous $K_2CO_3$ (14.21 g) and N,N-dimethyl thiocarbamoyl chloride (10.2 g) were added. The mixture was stirred at room temperature for 5¾ hours, then poured into water and left to stand overnight. The resultant solid was filtered off, washed well with water and dried at 50 ° *in vacuo* to give a yellow crystalline solid, 38.93 g.

MS m/e 280 ($M^+$, 75%), 278 (100%).

b) S-(2-Bromo-5-fluorophenyl)-N,N-dimethyl-thiocarbamate

The product of step a) (38.76 g) in N,N-diethyl aniline (150 ml) was heated to reflux for 5 hours. The mixture was allowed to cool overnight, then acidified with ice cold dilute HCl. The resultant cream precipitate was filtered off, washed well with dilute HCl and taken up in $CH_2Cl_2$. Drying and evaporation gave the sub-title compound as a cream solid which was dried at 50 ° *in vacuo* for 3 hours, 38.08 g.

MS m/e 278/280 ($M^+$).

c) 1-Bromo-4-fluoro-2-methylthiobenzene

A mixture of the product of step b) (37.09 g), 10% sodium hydroxide (358 ml) and methanol (500 ml) was heated to reflux under $N_2$ for 2 hours. The mixture was allowed to cool then evaporated to dryness. The residue was dissolved in water and extracted into $CH_2Cl_2$. The aqueous layer was acidified with conc. HCl and the resultant mixture extracted into ethyl acetate, washed ($H_2O$), dried and evaporated to leave a pale yellow oil, 22.78 g. This oil (22.78 g) in dry dimethylformamide (300 ml) was treated with anhydrous $K_2CO_3$ (18.50 g) and the mixture cooled in ice. Methyl iodide (17.67 g) in dry dimethylformamide (100 ml) was added dropwise with stirring under $N_2$. The mixture was stirred at room temperature for 1¼ hours then

poured into water and left to stand overnight. The resultant solid was filtered off, washed well with water, taken up in $CH_2Cl_2$, dried and evaporated to leave the sub-title compound as a cream solid, 21.71 g.

MS m/e 220/222 ($M^+$, 100%).

d) 2-(4-Fluoro-2-methylthiophenyl)-4-methoxy-pyridine

Magnesium turnings (1.07 g) in dry tetrahydrofuran (30 ml) and a crystal of iodine were stirred under $N_2$. The product of step c) (9.69 g) in dry tetrahydrofuran (60 ml) was added dropwise with stirring under $N_2$. The mixture was then heated to reflux for about 1½ hours.

The Grignard reagent thus prepared was added in one portion to a stirred solution of 4-methoxypyridine (4.78 g) in dry tetrahydrofuran (100 ml) cooled to -60°. The whole was stirred for 10 minutes then phenyl chloroformate (6.87 g) was added dropwise. The mixture was stirred at -60 ° for 30 minutes then allowed to warm to room temperature. After stirring for 1 hour 55 minutes, the mixture was quenched with water, extracted into ether, washed with water then brine, dried and evaporated to leave a yellow oil, 15.97 g. This oil was taken up in dry toluene (200 ml) and was treated dropwise, at room temperature with stirring, with a solution of o-chloranil (10.79 g) in glacial acetic acid (200 ml). The mixture was stirred at room temperature for 19 hours, then poured into ice water and made strongly basic with NaOH solution. After about 15 minutes, the mixture was filtered through kieselguhr and the residue washed through with ethyl acetate. The layers were separated. The organic layer was washed with brine then dilute HCl, the aqueous extracts were combined and basified with NaOH solution and the resultant white solid filtered off, washed with water, taken up in $CH_2Cl_2$, dried and evaporated to leave the sub-title compound as a white solid which was dried at 50° *in vacuo*, 5.73 g.

MS m/e 249 ($M^+$, 5%), 234 (100%).

e) 2-(4-Fluoro-2-methylsulphinylphenyl)-4-methoxy-pyridine

The product of step d) (5.80 g) in dichloromethane (160 ml), was cooled in an ice bath at about 5°, and m-chloroperbenzoic acid (85%, 4.73 g) in $CH_2Cl_2$ (200 ml) added. The mixture was stirred in an ice bath for 1¾ hours, then at room temperature for 1½ hours. The mixture was washed with saturated aqueous $NaHCO_3$, dried and evaporated to leave a yellow oily material which solidified. This was dissolved in a small volume of $CH_2Cl_2$ and chromatographed ($SiO_2$; petrol:ethyl acetate, 9:2, then $CH_2Cl_2$:ethyl acetate, 9:1) to give the sub-title compound as a white solid, 5.69 g.

mp 90-91°.

f) 2-[5-Fluoro-2-(4-methoxy-2-pyridinyl)-phenylthio]-1H-benzimidazole

The product of step e) (2.0 g) in dry $CH_2Cl_2$ (50 ml) was treated with trifluoroacetic anhydride (3.27 g) and the mixture heated to reflux for ½ hour. The whole was evaporated to dryness and treated with a degassed mixture of triethylamine (45 ml) and methanol (45 ml) then heated to reflux for ½ hour under $N_2$. The mixture was evaporated to dryness and treated with isopropanol (AR, 70 ml) and 2-chlorobenzimidazole (1.15 g), then heated at 80° under $N_2$ for 1½ hours. Sodium cyanoborohydride (0.48 g) and glacial acetic acid (100 ml) were added and the mixture heated at 80° for 1 hour. The mixture was basified with 10% NaOH, extracted into ethyl acetate, washed with brine (x4), dried and evaporated to leave a yellow oily foam, 2.75 g. This foam was chromatographed ($SiO_2$; $CH_2Cl_2$:ethyl acetate, 9:1) to give the sub-title product as a white foam.

mp 93-94°.

g) 2-[5-Fluoro-2-(4-methoxy-2-pyridinyl)-phenylsulphinyl]-1H-benzimidazole

The product of step f) (1.65 g) in $CH_2Cl_2$ (200 ml) was cooled in an ice-bath at about 5° and m-chloroperbenzoic acid (85%, 1.0 g) in $CH_2Cl_2$ (100 ml) added. The mixture was stirred in the ice bath for 1¾ hours, then washed with saturated aqueous $NaHCO_3$, dried and evaporated to give the title product as a white solid, 1.72 g.

mp 129- 130°.

## Example 2

## 2-[2-(4-Methoxy-2-pyridinyl)-6-methylphenylsulphinyl]-1H-benzimidazole

a) 2-Amino-3-methylbenzoic acid

A mixture of 3-methyl-2-nitrobenzoic acid (15 kg) and 5% palladium on charcoal (1.95 kg) in isopropanol (120 l) was hydrogenated at 75 psi pressure and 55-60° for 2-3 hours. The mixture was filtered hot and concentrated to approx. 20 l and treated with water (150 l). The solid product was isolated and dried to give the subtitle compound, 11.2 kg.

b) 3-Methyl-2-methylthiobenzoic acid

A mixture of the product of step a) (6 kg), water (54 l) and conc. HCl (9.6 l) was stirred at 0° and treated

with a solution of $NaNO_3$ (55.2 g) in water (240 ml). The reaction mixture was stirred for 2 hours at 0° and then added to a mixture of aqueous NaSMe (15%, 21 l) and KOH (21.3 kg) in water (15 l) at 55-60°. The reaction was stirred at approx. 55° for 1 hour and then 85° for 1 hour. The reaction was cooled to 15°, acidified with conc. HCl (36 l) and stirred overnight at 0°. The solid product was isolated and recrystallised from 40 l of 50% aqueous ethanol. The product was isolated and dried in vacuo at 50° to give the sub-title compound, 3.4 kg.

mp 112- 114°

c) 3-Methyl-2-methylthiobenzoyl chloride

The product from step b) (300 g) in $CH_2Cl_2$ (4.5 l) was treated with thionyl chloride (145 ml) and heated to reflux for 2½ hours. The mixture was filtered and concentrated to give a dark oil which was distilled under reduced pressure to give the sub-title compound, 300 g.

bp 124-130° at 1 mbar;

$^1H$ nmr δ $CDCl_3$ 7.52 (d, 1H), 7.43 (d, 1H), 7.32 (dd, 1H), 2.58 (s, 3H), 2.35 (s, 3H).

d) 1,4-Dihydro-2-(3-methyl-2-methylthiophenyl)-pyridine-4-one

To a stirred solution of tBuOK (100 g) in dry tetrahydrofuran (410 ml) at - 70° under nitrogen was added, dropwise, a solution of the product from step c) (81.5 g) and 4-methoxybut-3-en-2-one (63 ml) in tetrahydrofuran (410 ml) at <-60°. The reaction was left cold for ten minutes and then allowed to warm up to 0°. Water was added and the mixture extracted with $CH_2Cl_2$. The combined organic extracts were concentrated to an oil. The oil was dissolved in ethanol (1.43 l) and treated with ammonia (2.85 l) and ammonium acetate (200 g). This was heated to reflux for 19 hours and then concentrated to half volume. The concentrate was extracted with $CH_2Cl_2$ and the extracts concentrated to a syrup. This syrup was stirred in acetone (135 ml) at reflux until all the oil had converted to a solid. The mixture was cooled to room temperature and filtered to give the crude product. The crude product was slurried in methanol, the impurities filtered off and the filtrate concentrated then dried *in vacuo* at 50° to give the sub-title compound, 31 g.

MS thermospray (M+1) 232.

e) 4-Chloro-2-(3-methyl-2-methylthiophenyl)pyridine

The product from step d) (1.5 kg) was added to phosphoryl chloride (6 l) with cooling. The reaction was stirred overnight, under nitrogen, concentrated and the residue dissolved in dichloromethane. The solution was neutralised with strong caustic solution and the aqueous extracted with dichloromethane. The combined organics were dried and concentrated to give 1.6 kg of residue which crystallised on standing.

mp 37-39°;

MS thermospray (M+1) 250/252.

f) 4-Methoxy-2-(3-methyl-2-methylthiophenyl)pyridine

The product from step e) (575 g) in N-methyl-2-pyrrolidone (4 l) was stirred vigorously under nitrogen, treated with sodium methoxide (250 g) and the mixture heated at 60° for 4 hours. The reaction mixture was quenched with water and extracted with ethyl acetate. The extracts were dried and concentrated to give the sub-title compound containing approx. 10% of N-methyl-2-pyrolidone.

MS ($M^+$) 245, bp 230.

g) 4-Methoxy-2-(3-methyl-2-methylsulphinylphenyl)pyridine

The product of step f) (300 g after adjustment for N-methyl-2-pyrolidone) was dissolved in ethanol (1.9 l). The mixture was cooled to 0° and magnesium monoperoxyphthalate (83%, 4 lots of 92 g each dissolved in 500 ml water) was added, maintaining the temperature at 0-5°. Each individual solution of oxidant was made up shortly before being required. After addition was complete, the cooling bath was removed and the reaction left stirring for approx. 2 ½ hours. The reaction mixture was treated with sodium bicarbonate (120 g) dissolved in water (2.5 l) and the mixture cooled to 0°. The cooled mixture was stirred for 2 hours and the precipitated solid filtered off, washed with water and sucked dry. The isolated, solid product was dried *in vacuo* overnight at 60° to give the sub-title compound, 283 g.

MS ($M^+$) 261, bp 246.

h) 2-[2-(4-Methoxy-2-pyridinyl)-6-methylphenylthio]-1H-benzimidazole

Under nitrogen atmosphere, the product of step g) (75 g) was dissolved in $CH_2Cl_2$ (1.5 l) and treated with trifluoroacetic anhydride (44.7 ml) at 0°. The mixture was stirred at ambient temperature for ¾ hour. The mixture was then treated with isopropanol (0.9 l) and triethylamine (60 ml) and the stirred at ambient temperature for 1 hour. After this time further isopropanol (0.6 l) was added followed by acetic acid (82.25 ml), 2-chlorobenzimidazole (41.65 g) and sodium cyanoborohydride (4.51 g). The $CH_2Cl_2$ was distilled off from the reaction mixture and the cloudy reaction mixture heated at reflux for 27 hours. The reaction mixture was cooled and poured into water. Dichloromethane was added and the solution basified with NaOH solution (1N, 1.7 l). The organic layer was separated and the aqueous extracted with $CH_2Cl_2$. The combined organic layers were dried, filtered and concentrated to give a pale brown solid which was recrystallised

from isopropanol to give the sub-title compound, 59.7 g.

MS thermospray (M+1) 348.

i) 2-[2-(4-Methoxy-2-pyridinyl)-6-methylphenylsulphinyl]-1H-benzimidazole

The product from step h) (250 g) was dissolved in methanol (2 l), treated with lithium carbonate (79.9 g) and cooled to approx. 0°. To this stirred mixture was added a solution of magnesium monoperoxyphthalate (83%, 244 g) in methanol (0.5 l) maintaining the temperature at approx. 0°. The cold mixture was stirred for 2 hours, with light excluded, poured into cold water (10 l) and the resultant mixture stirred for 1 hour. The solid product was filtered off and dried *in vacuo* at 30°, over solid sodium hydroxide pellets. The crude solid was stirred in methanol (approx. 2 l) at room temperature. The turbid solution was filtered and the filtrate poured into vigorously stirred water (10 l) containing $NaHCO_3$ (84 g). The mixture was stirred for 1 hour at 1-2° and filtered. The damp solid was dried *in vacuo*, and over solid sodium hydroxide pellets, at 30°to give the title compound, 258 g.

MS (FAB) 364, bp 246.

j) Preparation of Enantiomers

A. (+)-2-[2-(4-Methoxy-2-pyridinyl)-6-methylphenylsulphinyl]-1H-benzimidazole

A mixture of the product of step h) (1.25 g) and (+)-(8,8-dichlorocamphorylsulphonyl)oxaziridine (1.1 g) in $CH_2Cl_2$ (11 ml) was stirred at room temperature, in the dark, for 39 days. The mixture was concentrated and the residue purified by preparative HPLC using an achiral column (Dynamax 60**Å**, 1% ethanol in $CHCl_3$ as eluent) to remove unreacted sulphide. The product sulphoxide was checked by chiral HPLC and was shown to comprise 95% of the title compound as the (+) enantiomer.

B. (-)-2-[2-(4-Methoxy-2-pyridinyl)-6-methylphenylsulphinyl]-1H-benzimidazole

This compound was prepared in analogous manner using (-)-(8,8-dichlorocamphorylsulphonyl) oxaziridine (1.1 g). The product sulphoxide was checked by chiral HPLC and was shown to comprise 90% of the title compound as the (-) enantiomer.

The following compounds were prepared by methods analogous to those above, via the intermediates indicated:

Example 3

2-[2-Fluoro-6-(4-methoxy-2-pyridinyl)phenylsulphinyl]-1H-benzimidazole

a) 3-Fluoro-2-methylthiobenzoic acid

$^1$H nmr δ $CDCl_3$ 7.84 (d, 1H), 7.87(dt, 1H), 7.29(t, 1H), 2.53 (d, 3H).

b) 3-Fluoro-2-methylthiobenzoyl chloride

$^1$H nmr δ $CDCl_3$ 7.73 (d, 1H), 7.37 (dt, 1H), 7.28 (dt, 1H), 2.52 (d, 3H).

c) 2-(3-Fluoro-2-methylthiophenyl)-1,4-dihydro-pyridine-4-one

MS FAB 236 ($M^+$+1).

d) 2-(3-Fluoro-2-methylthiophenyl)-4-methoxypyridine

MS 249 ($M^+$).

e) 2-(3-Fluoro-2-methylsulphinylphenyl)-4-methoxypyridine

mp 133°.

f) 2-[2-Fluoro-6-(4-methoxy-2-pyridinyl)phenylthio]-1H-benzimidazole

MS FAB 252 ($M^+$+1).

g) 2-[2-Fluoro-6(4-methoxy-3-pyridinyl)phenylsulphinyl]-1H-benzimidazole

mp 124°.

Example 4

2-[2-Chloro-6-(4-methoxy-2-pyridinyl)phenylsulphinyl]-1H-benzimidazole

a) 3-Chloro-2-methylthiobenzoic acid

mp 116-119°.

b) 3-Chloro-2-methylthiobenzoyl chloride

MS 221/223 ($M^+$).

c) 2-(3-Chloro-2-methylthiophenyl)-1,4-dihydro-pyridine-4-one
MS FAB 252/254 ($M^{+}$+1).

d) 2-(3-Chloro-2-methylthiophenyl)-4-methoxypyridine
MS FAB 226/228 ($M^{+}$+1).

e) 2-(3-Chloro-2-methylsulphinylphenyl)-4-methoxypyridine
mp 146-149°.

f) 2-[2-Chloro-6-(4-methoxy-2-pyridinyl)phenylthio]-1H-benzimidazole
mp 230°.

g) 2-[2-Chloro-6-(4-methoxy-2-pyridinyl)phenylsulphinyl]-1H-benzimidazole
mp 160° (decomposes).

Example 5

Tablet Formulation

| | | % w/w |
|---|---|---|
| Compound of Example 2 | | 1.0% |
| Microcrystalline cellulose (Avicel® PH102) | | 20.0% |
| Sorbitol Instant® | | 15.0% |
| Magnesium stearate | | 1.0% |
| Sodium carboxymethylcellulose (Ac-Di-Sol®) | | 2.5% |
| Spray dried lactose | to | 100% |

The compound of Example 2 was blended with the remaining excipients to produce a homogeneous mixture which was then compressed into round 200 mg tablets.

Example 6

Enteric Coating for Tablet Formulation

| | | % w/w |
|---|---|---|
| Methacrylic acid copolymer type B | | 6.5% |
| Dibutyl phthalate | | 1.63% |
| Purified water | | 1.5% |
| Isopropyl alcohol | to | 100% |

The above solution was applied to the tablets of Example 5 using a spray gun. The solvents were allowed to dry leaving an enteric film of 3 to 7 mg/$cm^2$ of tablet surface area.

**Claims**

**1.** A compound of formula I,

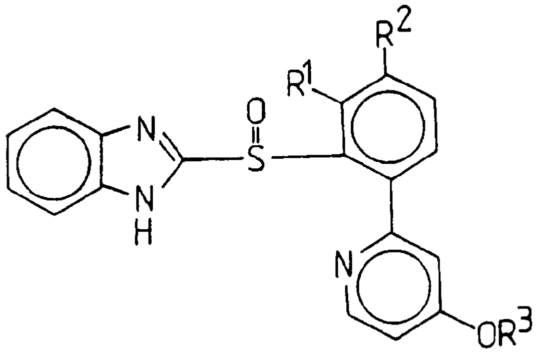

I

wherein one of $R^1$ and $R^2$ represents halogen or alkyl $C_{1-6}$, and the other represents hydrogen, $R^3$ represents alkyl $C_{1-6}$,
and pharmaceutically acceptable salts thereof.

2. A compound according to Claim 1, wherein $R^1$ represents halogen or alkyl $C_{1-6}$.

3. A compound according to Claim 1 or 2, wherein $R^1$ represents methyl.

4. A compound according to any one of the preceding claims, wherein $R^3$ represents methyl.

5. A compound according to Claim 1 which is,
2-[5-Fluoro-2(4-methoxy-2-pyridinyl)-phenylsulphinyl]-1H-benzimidazole,
(+)-2-[2-(4-Methoxy-2-pyridinyl)-6-methylphenylsulphinyl]-1H-benzimidazole,
(-)-2-[2(4-Methoxy-2-pyridinyl)-6-methylphenylsulphinyl]-1H-benzimidazole,
2-[2-Fluoro-6-(4-methoxy-2-pyridinyl)phenylsulphinyl]-1H-benzimidazole, or
2-[2-Chloro-6-(4-methoxy-2-pyridinyl)phenylsulphinyl]-1H-benzimidazole,
or a pharmaceutically acceptable salt of any one thereof.

6. A compound of formula I, as defined in Claim 1, or a pharmaceutically acceptable salt thereof, for use as a pharmaceutical.

7. A pharmaceutical composition comprising a compound of formula I as defined in Claim 1, or a pharmaceutically acceptable salt thereof, in admixture with a pharmaceutically acceptable adjuvant, diluent or carrier.

8. The use of a compound of formula I as defined in Claim 1, or a pharmaceutically acceptable salt thereof, in the manufacture of a medicament for the treatment or prophylaxis of a condition in which gastric acid has a pathological role.

9. A process for the production of a compound of formula I as defined in Claim 1, or a pharmaceutically acceptable salt thereof which comprises selective oxidation of a corresponding compound of formula II,

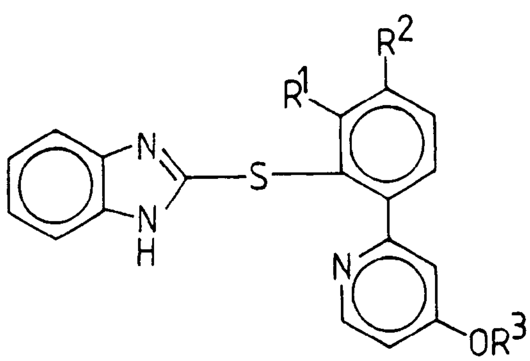

II

in which $R^1$, $R^2$ and $R^3$ are as defined in Claim 1, and where necessary or desired converting the

resulting compound of formula I to a pharmaceutically acceptable salt thereof, or vice versa.

**10.** A compound of formula II,

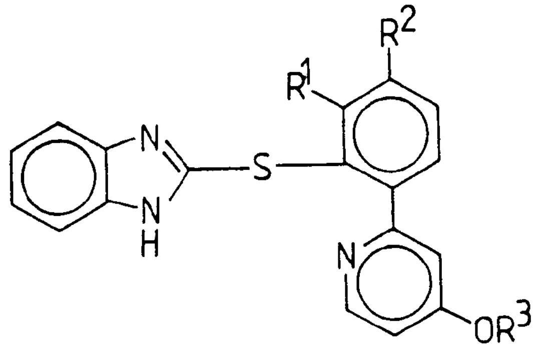

II

in which $R^1$, $R^2$ and $R^3$ are as defined in Claim 1.

European Patent Office

# EUROPEAN SEARCH REPORT

Application Number

EP 92 30 6426

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X | EP-A-0 220 053 (FISONS PLC)<br>* page 2, line 12 - line 39 *<br>* page 2, line 55 - line 59 *<br>* page 3, line 3 *<br>* page 3, line 20 - line 43 *<br>* page 6, line 26 - line 53 *<br>--- | 1-10 | C07D401/12<br>A61K31/415<br>A61K31/44 |
| D,X | EP-A-0 262 845 (FISONS PLC)<br>* page 1 - page 2, line 14 *<br>* page 10, line 30 - page 11, line 2 *<br>----- | 1-10 | |

TECHNICAL FIELDS SEARCHED (Int. Cl.5 )

C07D
A61K

The present search report has been drawn up for all claims

| Place of search | Date of completion of the search | Examiner |
|---|---|---|
| THE HAGUE | 13 NOVEMBER 1992 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)